# Europäisches Patentamt
# European Patent Office
# Office européen des brevets

(19)

(11) Veröffentlichungsnummer : **0 435 070 B1**

(12) **EUROPÄISCHE PATENTSCHRIFT**

(45) Veröffentlichungstag der Patentschrift :
**09.03.94 Patentblatt 94/10**

(51) Int. Cl.⁵ : **C07C 31/20**, C07C 29/141,
C07C 29/80

(21) Anmeldenummer : **90123910.3**

(22) Anmeldetag : **12.12.90**

(54) Verfahren zur Herstellung von 2,2-Dimethylpropandiol-(1,3).

(30) Priorität : **23.12.89 DE 3942792**

(43) Veröffentlichungstag der Anmeldung :
**03.07.91 Patentblatt 91/27**

(45) Bekanntmachung des Hinweises auf die
Patenterteilung :
**09.03.94 Patentblatt 94/10**

(84) Benannte Vertragsstaaten :
**AT BE CH DE ES FR GB IT LI NL SE**

(56) Entgegenhaltungen :
EP-A- 0 278 106
DD-A- 273 434
DE-A- 2 653 096
DE-A- 3 638 496
DE-B- 2 045 668

(73) Patentinhaber : HOECHST
AKTIENGESELLSCHAFT
D-65926 Frankfurt (DE)

(72) Erfinder : Dämbkes, Georg, Dr. Dipl.-Chem.
Nibelungenstrasse 65
W-4220 Dinslaken (DE)
Erfinder : Lappe, Peter, Dr. Dipl.-Chem.
Eickenhof 34
W-4220 Dinslaken (DE)
Erfinder : Thönnessen, Franz, Dr. Dipl.-Chem.
Lützowstrasse 49
W-4200 Oberhausen 11 (DE)
Erfinder : Springer, Helmut, Dipl.-Chem.
Borbeckerstrasse 19
W-4200 Oberhausen 11 (DE)

EP 0 435 070 B1

**Beschreibung**

Die Erfindung betrifft ein Verfahren zur Herstellung von 2,2-Dimethylpropandiol-(1,3) aus Isobutyraldehyd und Formaldehyd unter Verwendung von tertiären Aminen als Katalysator, Hydrierung des Reaktionsgemisches und anschließende Destillation des Hydrierproduktes nach Zusatz von Formaldehyd.

Es ist bekannt, aus Isobutyraldehyd und Formaldehyd durch Aldoladdition 2,2-Dimethyl-3-hydroxypropanal herzustellen und diesen Oxyaldehyd anschließend zum entsprechenden Diol zu hydrieren. Als Katalysatoren für die Aldoladdition finden basisch reagierende Verbindungen Verwendung. Weit verbreitet ist der Einsatz von Alkalihydroxiden, Erdalkalihydroxiden und Alkalicarbonaten. Daneben gelangen auch Amine, insbesondere tertiäre Mono- oder Polyamine, z.B. Diamine, zur Anwendung. Ein derartiges Verfahren ist z.B. in der DE 19 57 591 B2 beschrieben. Zur Herstellung von 2,2-Dimethylpropandiol-(1,3) setzt man Isobutyraldehyd und Formaldehyd in Gegenwart von tertiären Aminen um und hydriert das anfallende Reaktionsgemisch mit Wasserstoff. Als geeignet werden u.a. folgende Amine genannt: Trimethyl-, Triethyl-, Methyl-diethyl-, Methyl-diisopropylamin, Tributylamin.

Der Einsatz von Aminen als Additionskatalysatoren hat den Vorteil, daß aus Isobutyraldehyd und Formaldehyd Nebenprodukte, die bei Verwendung anderer basischer Katalysatoren gebildet werden, nicht oder nur in untergeordneter Menge entstehen. Dagegen muß der vollständigen Entfernung der Amine Beachtung geschenkt werden, weil mit Aminen auch nur in geringer Menge verunreinigtes Diol für viele Verwendungszwecke ungeeignet ist. Die Herstellung von reinem 2,2-Dimethylpropandiol-(1,3) verlangt daher einen sehr hohen Trennaufwand.

Nach der DE 36 44 675 A1 gelingt es, das tertiäre Amin aus dem Diol vollständig zu entfernen, wenn man als Additionskatalysator Tri-n-propylamin einsetzt und das Rohprodukt in Gegenwart von Isobutanol destilliert. Diese Arbeitsweise beseitigt aber nicht oder nur unvollständig, primäre und sekundäre Amine. Beide Verbindungstypen begleiten aus dem Herstellungsprozeß das tertiäre Amin oder entstehen aus dem als Katalysator verwendeten tertiären Amin bei der Hydrierung des Additionsproduktes. Durch Reaktion der primären und sekundären Amine mit Isobutyraldehyd und/oder Formaldehyd bilden sich im Verlauf der Umsetzung basisch reagierende Produkte, die aus dem 2,2-Dimethylpropandiol-(1,3) nicht oder nur teilweise abgetrennt werden können.

Es bestand daher die Aufgabe ein Verfahren zu entwickeln, das eine sichere und vollständige Beseitigung der basischen Komponenten aus dem 2,2-Dimethylpropandiol-(1,3) ermöglicht.

Erfindungsgemäß wird diese Aufgabe gelöst durch ein Verfahren zur Herstellung von 2,2-Dimethylpropandiol-(1,3) durch Addition von Isobutyraldehyd und Formaldehyd in Gegenwart von tertiären Aminen als Katalysator, Hydrierung des Reaktionsgemisches und anschließende Destillation des Hydrierproduktes, wobei die Destillation nach Zusatz von Formaldehyd erfolgt.

Das neue Verfahren liefert sehr reines 2,2-Dimethylpropandiol-(1,3), das basisch reagierende Umwandlungsprodukte primärer oder sekundärer Amine nur noch in solchen Mengen enthält, die einer Weiterverarbeitung des Diols nicht entgegenstehen. Wird dafür Sorge getragen, daß auch als Katalysator verwendetes tertiäres Amin z.B. destillativ vollständig oder zumindest annähernd vollständig entfernt wird, dann erhält man ein 2,2-Dimethylpropandiol-(1,3), das in allen bekannten Anwendungsgebieten eingesetzt werden kann.

Nach der beanspruchten Arbeitsweise läßt man in der 1. Stufe Formaldehyd und Isobutyraldehyd miteinander reagieren. Die Ausgangsstoffe können im molaren Verhältnis eingesetzt werden, jedoch ist es auch möglich, einen der beiden Reaktionspartner im Überschuß anzuwenden. Formaldehyd setzt man zweckmäßig als wäßrige Lösung ein, ihr Aldehydgehalt beträgt üblicherweise 30 bis 49 Gew.-%. Die Reaktion erfolgt bei Temperaturen zwischen 20 und 130°C, es hat sich bewährt, bei 80 bis 95°C zu arbeiten. Im allgemeinen wird die Umsetzung bei Normaldruck durchgeführt, jedoch kann man auch erhöhten Druck anwenden. Die Zugabe von Lösungsmitteln ist nicht erforderlich.

Als Katalysatoren werden tertiäre Amine verwendet. Sie sind im Reaktionsgemisch in einer Menge von 1 bis 20, vorzugsweise 2 bis 12 Mol.-%, bezogen auf Isobutyraldehyd, enthalten. Das Verfahren ist nicht an die Verwendung eines bestimmten tertiären Amins als Katalysator gebunden. Die Auswahl des Amins wird vor allem durch die Forderung bestimmt, es durch konventionelle Maßnahmen möglichst quantitativ aus dem Reaktionsprodukt entfernen zu können. Besonders bewährt hat es sich, Tri-n-propylamin als Katalysator einzusetzen, weil es in einfacher Weise nach dem Verfahren der DE 36 44 675 A1 als Azeotrop mit Wasser und i-Butanol aus dem 2,2-Dimethylpropandiol-(1,3) abdestilliert werden kann.

Die praktische Durchführung der Additionsreaktion erfolgt in einem Rührkessel oder in einem Reaktionsrohr, das zur besseren Durchmischung der Reaktanten mit Füllkörpern beschickt ist. Die Umsetzung verläuft exotherm, sie kann durch Erhitzen beschleunigt werden. Das anfallende Reaktionsgemisch wird ohne vorherige Trennung in seine Bestandteile oder Abtrennung einzelner Komponenten katalytisch hydriert. Die Anlagerung von Wasserstoff kann in der Gasphase oder auch in flüssiger Phase erfolgen. Als Katalysatoren eigenen sich insbesondere Nickel-Trägerkatalystoren, die gegebenenfalls noch weitere aktive Metalle wie Kupfer oder Chrom und darüber hinaus Aktivatoren enthalten können.

Das Hydrierprodukt kann unmittelbar, d.h. ohne zusätzliche Reinigungsoperationen weiterverarbeitet werden. Lediglich bei Verwendung von suspendierten Hydrierkatalysatoren empfiehlt es sich, letzte Katalysatorreste durch Filtration zu entfernen. Ein typisches Hydrierprodukt weist etwa folgende Zusammensetzung auf (die %-Angaben beziehen sich jeweils auf das Gemisch):

| | |
|---|---|
| 2,2-Dimethylpropandiol-(1,3) | 30 bis 74 Gew.-% |
| Isobutanol | 20 bis 60 Gew.-% |
| tertiäres Amin | 5 bis 15 Gew.-% |
| primäres, sekundäres Amin | 0,02 bis 0,2 Gew.-% |
| sonstige Bestandteile | 0,1 bis 5 Gew.-% |

Abhängig von der individuellen Ausgestaltung des Additions- und des Hydrierschrittes sind von den obigen Angaben abweichende Zusammensetzungen des Hydrierproduktes möglich. Sie beeinflussen die Anwendbarkeit des neuen Verfahrens jedoch nicht.

Erfindungsgemäß wird das Hydrierprodukt nach Zusatz von Formaldehyd destilliert. Die zuzusetzende Aldehydmenge richtet sich nach dem Gehalt des Einsatzgemisches an primärem und sekundärem Amin. Dieser Gehalt ist analytisch, z.B. durch Gaschromatographie zu bestimmen. Je mol Amin setzt man 2 bis 100, vorzugsweise 20 bis 50 mol Formaldehyd ein. Zweckmäßig gelangt er als wäßrige Lösung mit einem Gehalt von 30 bis 49 Gew.-% Formaldehyd (bezogen auf die Lösung) zum Einsatz. Das üblicherweise in diesen Lösungen zur Stabilisierung enthaltene Methanol (zumeist in einem Anteil von etwa 1 Gew.-%) stört ihre Anwendbarkeit nicht. Mit gleich gutem Erfolg wie die wäßrige Lösung kann Formaldehyd auch in polymerer Form als Paraformaldehyd eingesetzt werden.

Die Zugabe des Formaldehyds erfolgt in das auf 40 bis 160°C, vorzugsweise 55 bis 130°C erhitzte Hydrierprodukt. Man läßt die Reaktanten 0,01 bis 24 h, vorzugsweise 0,1 bis 8 h und insbesondere 0,5 bis 4 h aufeinander einwirken, wobei drucklos oder unter Drücken bis zu 1 MPa gearbeitet werden kann, bevorzugt werden Drücke von 10 bis 50 kPa.

In dem, wie vorstehend beschrieben, behandelten Hydrierprodukt lassen sich primäre und sekundäre Amine nur noch in untergeordneten Mengen nachweisen. Es kann zur Reinigung in bekannter Weise unter normalem oder unter vermindertem Druck destilliert werden. Gewöhnlich setzt man kontinuierlich arbeitende Fraktionierkolonnen mit 40 bis 120, vorzugsweise 50 bis 70 theoretischen Böden ein. Die Kolonne kann mit Seitenabzügen zur Abnahme der verschiedenen Bestandteile des Gemisches, insbesondere des tertiären Amins und des Isobutanols sowie von Azeotropen, die sich ausbilden können, ausgestattet sein.

Die basisch reagierenden, aus den primären und sekundären Aminen gebildeten Verbindungen destillieren zusammen mit dem als Katalysator eingesetzten tertiären Amin über. Diese Fraktion kann, ohne sie vorher in ihre Bestandteile zu trennen, in den Prozeß zurückgeführt und erneut als Katalysator genutzt werden.

Verwendet man, wie bereits weiter oben beschrieben, Tri-n-propylamin als Katalysator, kann die Destillation in einer mit zwei Seitenabzügen versehenen Kolonne durchgeführt werden. An der unteren Seitenabnahme werden Tri-n-propylamin, Restmengen Isobutanol und die basischen Verunreinigungen abgenommen. An der oberen Seitenabnahme zieht man ein Zweiphasensystem ab, dessen organische Phase hauptsächlich aus Isobutanol besteht. Am Kopf der Kolonne fällt Methanol an, das zusammen mit leicht siedenden Nebenprodukten ausgeschleust wird. Reaktionswasser und das restliche Methanol werden über die Wasserphase des oberen Seitenabzugs abgetrennt.

Das neue Verfahren wird durch die nachstehenden Beispiele näher erläutert.

Beispiele

In den folgenden Beispielen gelangte ein Gemisch der Zusammensetzung

| | |
|---|---|
| 2,2-Dimethylpropandiol-(1,3) | 36,0 Gew.-% |
| Isobutanol | 54,4 Gew.-% |
| Tri-n-propylamin | 7,88 Gew.-% |
| Mono- und Di-n-propylamin | 0,04 Gew.-% |
| sonstige Bestandteile | 1,68 Gew.-% |

zum Einsatz. Es wurde durch Hydrierung des Additionsproduktes aus Formaldehyd und Isobutyraldehyd in Isobutanol als Lösungsmittel und in Gegenwart von Tri-n-propylamin als Katalysator erhalten.

Dem Produkt wurden zur Abtrennung des Mono- und des Di-n-propylamins unterschiedliche Mengen Formalin zugesetzt. Die Ergebnisse der Untersuchungen sind in der folgenden Übersicht zusammengestellt.

| Mol HCHO je (37 %ig) Mol MnPA/DnPA* | Temp. (°C) | t (h) | p (MPa) | GC-Analyse MnPA/DnPA* (%) |
|---|---|---|---|---|
| 15 | 94 | 1 | – | 0,001 |
| 10 | 94 | 1 | – | 0,002 |
| 5 | 94 | 1 | – | 0,018 |
| 3 | 94 | 1 | – | 0,023 |
| 10 | 130 | 1 | 0,32 | 0,001 |
| 5 | 130 | 1 | 0,32 | 0,02 |

\* MnPA = Mono-n-propylamin
DnPA = Di-n-propylamin

Das gereinigte 2,2-Dimethylpropandiol-(1,3) wies eine Alkalität von <5 ppm N auf.

**Patentansprüche**

1. Verfahren zur Herstellung von 2,2-Dimethylpropandiol-(1,3) durch Addition von Isobutyraldehyd und Formaldehyd in Gegenwart von tertiären Aminen als Katalysator, Hydrierung des Reaktionsgemisches und anschließende Destillation des Hydrierproduktes, dadurch gekennzeichnet, daß die Destillation nach Zusatz von Formaldehyd erfolgt.

2. Verfahren nach Anspruch 1, dadurch gekennzeichnet, daß je mol im Hydrierprodukt enthaltenes primäres und sekundäres Amin 2 bis 100, vorzugsweise 20 bis 50 mol Formaldehyd eingesetzt wird.

3. Verfahren nach Anspruch 1 oder 2, dadurch gekennzeichnet, daß Formaldehyd als wäßrige Lösung oder als Paraformaldehyd verwendet wird.

4. Verfahren nach einem oder mehreren der Ansprüche 1 bis 3, dadurch gekennzeichnet, daß Formaldehyd in das auf 40 bis 160, vorzugsweise 55 bis 130°C erhitzte Hydrierprodukt gegeben wird.

5. Verfahren nach einem oder mehreren der Ansprüche 1 bis 4 dadurch gekennzeichenet, daß man Formaldehyd und Hydrierprodukt 0,01 bis 24 h vorzugsweise 0,1 bis 8 h und insbesondere 0,5 bis 4 h aufeinander einwirken läßt.

6. Verfahren nach einem oder mehreren der Ansprüche 1 bis 5, dadurch gekennzeichnet, daß als Katalysator Tri-n-propylamin eingesetzt wird.

7. Verfahren nach einem oder mehreren der Ansprüche 1 bis 6, dadurch gekennzeichnet, daß die Reaktion zwischen Formaldehyd und Isobutyraldehyd bei Temperaturen zwischen 20 und 130, vorzugsweise 80 bis 95°C erfolgt.

**Claims**

1. A process for the preparation of 2,2-dimethylpropane-1,3-diol by an addition reaction between isobutyraldehyde and formaldehyde in the presence of tertiary amines as the catalyst, hydrogenation of the reaction mixture and subsequent distillation of the hydrogenation product, which comprises carrying out the

4

**EP 0 435 070 B1**

distillation after addition of formaldehyde.

2. The process as claimed in claim 1, wherein 2 to 100, preferably 20 to 50 mol of formaldehyde is employed per mol of primary and secondary amine contained in the hydrogenation product.

3. The process as claimed in claim 1 or 2, wherein the formaldehyde is used as an aqueous solution or as paraformaldehyde.

4. The process as claimed in one or more of claims 1 to 3, wherein the formaldehyde is added to the hydrogenation product which has been heated up to 40 to 160, preferably 55 to 130°C.

5. The process as claimed in one or more of claims 1 to 4, wherein the formaldehyde and hydrogenation product are allowed to act on one another for 0.01 to 24 hours, preferably 0.1 to 8 hours and in particular 0.5 to 4 hours.

6. The process as claimed in one or more of claims 1 to 5, wherein tri-n-propylamine is employed as the catalyst.

7. The process as claimed in one or more of claims 1 to 6, wherein the reaction between the formaldehyde and isobutyraldehyde is carried out at temperatures between 20 and 130, preferably 80 and 95°C.

**Revendications**

1. Procédé de préparation du 2,2-diméthylpropanediol-1,3 par addition de l'aldéhyde isobutyrique et du formaldéhyde en présence d'amines tertiaires servant de catalyseurs, hydrogénation du mélange de réaction, puis distillation du produit d'hydrogénation, caractérisé en ce que la distillation est réalisée après adjonction de formaldéhyde.

2. Procédé selon la revendication 1, caractérisé en ce que l'on utilise de 2 à 100, de préférence de 20 à 50 mol de formaldéhyde par mole d'amines primaires et secondaires contenues dans le produit d'hydrogénation.

3. Procédé selon la revendication 1 ou 2, caractérisé en ce que le formaldéhyde est utilisé à l'état de solution aqueuse ou à l'état de paraformaldéhyde.

4. Procédé selon une ou plusieurs des revendications 1 à 3, caractérisé en ce que le formaldéhyde est ajouté au produit d'hydrogénation chauffé à une température de 40 à 160, de préférence de 55 à 130°C.

5. Procédé selon une ou plusieurs des revendications 1 à 4, caractérisé en ce qu'on laisse agir le formaldéhyde et le produit d'hydrogénation entre eux pendant une durée de 0,01 à 24 h, de préférence de 0,1 à 8 h et plus spécialement de 0,5 à 4 h.

6. Procédé selon une ou plusieurs des revendications 1 à 5, caractérisé en ce que le catalyseur utilisé est la tri-n-propylamine.

7. Procédé selon une ou plusieurs des revendications 1 à 6, caractérisé en ce que la réaction entre le formaldéhyde et l'aldéhyde isobutyrique est effectuée à des températures de 20 à 130, de préférence de 80 à 95°C.

5